(19) Europäisches Patentamt
European Patent Office
Office européen des brevets

(11) Veröffentlichungsnummer: **0 253 922**
**A1**

(12) # EUROPÄISCHE PATENTANMELDUNG

(21) Anmeldenummer: **86115246.0**

(51) Int. Cl.⁴: **C07C 33/14** , C07C 29/76

(22) Anmeldetag: **04.11.86**

(43) Veröffentlichungstag der Anmeldung: **27.01.88 Patentblatt 88/04**

(84) Benannte Vertragsstaaten: **CH DE LI**

(71) Anmelder: **Dragoco Gerberding & Co. GmbH**
**Dragocostrasse 1**
**D-3450 Holzminden(DE)**

(72) Erfinder: **Brunke, Ernst-Joachim, Dr.**
**Holbeinplatz 6**
**D-3450 Holzminden(DE)**
Erfinder: **Köster, Frank-Hinrich, Dr.**
**Luise-Hensel-Strasse 11**
**D-3470 Höxter 1 (Lüchtringen)(DE)**

(74) Vertreter: **Eikenberg & Brümmerstedt**
**Patentanwälte**
**Schackstrasse 1**
**D-3000 Hannover 1(DE)**

(54) **Verfahren zur Reinigung von Alpha-Bisabolol.**

(57) α-Bisabolol besitzt ausgesprochene geruchsfixierende Eigenschaften, und deshalb ist die Abtrennung geruchlich störender Begleitstoffe aus einem Rohprodukt (das natürlichen oder synthetischen Ursprungs sein kann) sehr schwierig. Die üblichen Reinigungsoperationen wie Destillation, Adsorption an Aktivkohle o. dgl. versagen, so daß bislang verhältnismäßig aufwendige chemische Behandlungen erforderlich waren.

Das jetzt vorgeschlagene Reinigungsverfahren besteht darin, daß eine Lösung des rohen α-Bisabolols in einem inerten Lösungsmittel mit einem Siedepunkt zwischen 40 und 200 °C, vorzugsweise 60 - 150 °C, über ein Austauscherharz filtriert, anschließend vom Lösungsmittel befreit und dann ggfs. weiteren Reinigungsoperationen unterworfen wird. Das Lösungsmittel ist dabei zweckmäßig ein aliphatischer Alkohol mit bis zu 6 C-Atomen, vorzugsweise Isopropanol, der in einer Menge von 10 bis 1000 Gew.%, vorzugsweise 50 - 200 Gew.%, bezogen auf das rohe α-Bisabolol, eingesetzt wird. Als Austauscherharz ist ein hochpolymeres vernetztes Polykondensations-oder Polymerisationsharz bevorzugt, dessen aktive Gruppen wahlweise saure Gruppen, vorzugsweise Sulfonsäure-, Carboxylat-oder Phenolgruppen oder basische Gruppen, vorzugsweise ein-oder mehrfach alkylierte Aminogruppen sind, wobei als Einsatzmenge 5 - 100 Gew.%, vorzugsweise 10 - 50 Gew.% Harz, bezogen auf das rohe α-Bisabolol, ausreichend sind.

Dieses Reinigungsverfahren ist überraschend gut wirksam und zeichnet sich im Vergleich zu den chemischen Behandlungen durch große Einfachheit, geringen Aufwand an Geräten, Arbeit und Hilfsstoffen sowie eine sehr niedrige Umweltbelastung aus.

EP 0 253 922 A1

## Verfahren zur Reinigung von α-Bisabolol

Der Sesquiterpenalkohol α-Bisabolol besitzt zwei Asymmetriezentren und kann daher in vier stereoisomeren Formen vorkommen. Alle vier Stereoisomere sind als Pflanzeninhaltsstoffe in ätherischen Ölen nachgewiesen worden.

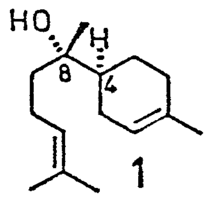

1

(-)-α-Bisabolol [4S, 8S]

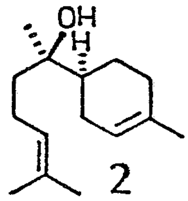

2

(-)-epi-α-Bisabolol [4S, 8R]

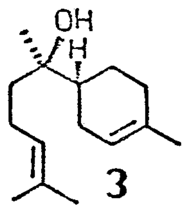

3

(+)-α-Bisabolol [4R, 8R]

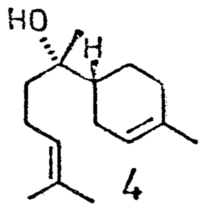

4

(+)-epi-α-Bisabolol [4R, 8S]

So ist z. B. (-)-α-Bisabolol **1** ein Inhaltsstoff von *Matricaria chamomilla* (M.A. Schwartz, G.C. Swanson J. Org. Chem. 44, 953 (1979)) und *Vanillosmopsis erythropappa* (O.R. Gottlieb, M.T. Magalh es, Perf. Essent. Oil. Rec. 1958, 711). Das zu **1** enantiomere (+)-α-Bisabolol **3** wurde im Pappelknospenöl *Populus balsamifera* (E. Flaskamp, G. Nonnenmacher, O. Isaac, Z. Naturforsch. 36b , 114 (1981); F. Sorm, M. Vran , V. Herout, Chem. Listy 46 , 364 (1952)) und in *Atalantia monophylla correa* (J.D. Shringarpure, B.K. Sabata, Ind. J. Chem. 13 , 24 (1975)) nachgewiesen. Das C-8 Epimere von **1**, das (-)-epi-α-Bisabolol **2** ist Bestandteil des ätherischen Öles von *Myoporum crassifolium* (K.G. O'Brien, A.R. Penfold, M.D. Sutherland, R.L. Werner, Australian J. Chem. 7, 298 (1954)). Kürzlich wurde auch das vierte Stereoisomere, nämlich das (+)-epi-α-Bisabolol **4** in dem ätherischen Öl von *Salvia stenophylla* entdeckt (E.-J. Brunke, F.-J. Hammerschmidt in A. Baerheim Svendsen und J.J.C. Scheffer (Hrsgb.), Essential Oils and Aromatic Plants, Martinus Nijhoff/Dr. W. Junk Publishers Dordrecht 1985, The Netherlands, 145 ff).

Reines α-Bisabolol ist eine farblose Flüssigkeit mit nur schwachem Eigengeruch, welches wegen seiner vorteilhaften Wirkungen auf die Haut (O. Isaac, H. Schneider, E. Eggenschwiller, Dtsch. Apotheker Ztg. 108, 293 (1968)) als Wirkstoff Eingang in kosmetischen Zubereitungen gefunden hat. Zudem besitzt es ausgesprochene geruchsfixierende Eigenschaften, so daß es auch vorteilhaft als Fixateur von Riechstoffen in der Kosmetik verwendet werden kann (DE-PS 10 81 170). Das zu diesem Zweck verwendete α-Bisabolol kann synthetischen oder natürlichen Ursprungs sein, wobei die kosmetische Industrie in zunehmendem Ausmaß das natürliche α-Bisabolol verlangt, weil ein steigendes Interesse an natürlichen Kosmetika besteht. Aber gerade wegen der geruchsfixierenden Eigenschaften des α-Bisabolols hat sich die Gewinnung von reinem, also farb-und geruchsneutralem α-Bisabolol aus pflanzlichen Quellen als außerordentlich schwierig erwiesen, denn das α-Bisabolol ist in den ätherischen Ölen der Pflanzen in der Regel mit stark riechenden Substanzen vergesellschaftet, die entweder nicht oder nur sehr schwer abtrennbar sind und sich bereits in sehr geringen Konzentrationen störend bemerkbar machen. Ähnliche Schwierigkeiten ergeben sich übrigens auch bei synthetischem α-Bisabolol, bei dem die möglicherweise störenden Begleitstoffe verfahrensbedingt sind.

In der DE-PS 23 17 583 wird unter Hinweis auf die Nicht-Eignung üblicher Verfahren (z.B. Hochvakuumdestillation) ein aufwendiges chemisches Verfahren zur Reinigung von rohem pflanzlichen α-Bisabolol beschrieben. Hierbei wird das in einem Lösungsmittel gelöste Rohprodukt mit Alkalihydroxiden, Alkalialkoholaten, Alkalicarbonaten, Alkalibicarbonaten, Erdalkalioxiden, Erdalkalihydroxiden oder Aluminiumhydroxid bei 0° - 50°C behandelt (z.B. geschüttelt) und danach wieder vom Lösungsmittel befreit. Eine während dieser Behandlung auftretende Emulsionsbildung kann durch weniger intensives Mischen oder durch Zugabe von

üblichen Antischaummitteln zurückgedrängt werden. Zusätzlich kann die Alkalibehandlung noch mit einer Aktivkohle-Behandlung kombiniert werden, und anschließend kann das Produkt auch noch einer Destillation unterworfen werden. Die Aktivkohle-Behandlung hat dabei nur den Zweck, noch weitere, geruchlich nicht relevante Begleitstoffe zu entfernen und die anschließende Destillation zu vereinfachen. Eine alleinige Aktivkohle-Behandlung reicht jedoch in keinem Falle aus, die geruchlich störenden Bestandteile zu entfernen, ebenso wie auch die anschließende Destillation nur der weiteren Reinigung des bereits von den Geruchsstoffen befreiten Produktes dient.

Die Reinheit des auf diese Weise gewonnenen α-Bisabolols ist befriedigend. Das Verfahren ist aber aufgrund der möglichen Emulsionsbildung im industriellen Maßstab nicht unproblematisch, und außerdem führt es zu einer zusätzlichen Salzbelastung industrieller Abwässer. Es besteht daher ein großer Bedarf an einem technisch einfacheren und umweltschonenden Verfahren. Ein solches Verfahren soll mit der Erfindung zur Verfügung gestellt werden.

Das erfindungsgemäße Verfahren ist sowohl bei aus Pflanzenmaterial isoliertem als auch durch Synthese gewonnenem rohen α-Bisabolol anwendbar und kennzeichnet sich dadurch, daß eine Lösung des rohen α-Bisabolols in einem inerten Lösungsmittel mit einem Siedepunkt zwischen 40 und 200 °C, vorzugsweise 60 - 150 °C über ein Austauscherharz filtriert, anschließend vom Lösungsmittel befreit und dann ggfs. weiteren Reinigungsoperationen unterworfen wird.

Die Erfindung beruht auf der Erkenntnis, daß zur Abtrennung von störenden Geruchssubstanzen aus rohem α-Bisabolol keine aufwendige chemische Behandlung erforderlich ist, sondern eine einfache Filtration über ein Austauscherharz - in der Regel mit einmaligem Durchlauf - völlig ausreicht. Diese Erkenntnis ist ausgesprochen überraschend und war in Anbetracht der bekannten Tatsache, daß mit Aktivkohle als üblichem Adsorbens selbst mit hohen Kohlekonzentrationen und langen Behandlungszeiten keine geruchliche Reinigung des α-Bisabolols gelingt, auch nicht vorhersehbar.

Als inertes Lösungsmittel kommen für das erfindungsgemäße Verfahren alle Lösungsmittel in Betracht, die sich unproblematisch wieder von dem α-Bisabolol z.B. durch Destillation abtrennen lassen. Bevorzugt sind dies polare Lösungsmittel wie niedrige aliphatische Alkohole mit bis zu 6 C-Atomen, vorzugsweise Isopropanol. Zweckmäßig wird das Lösungsmittel dabei in einer Menge von 10 - 1000 Gew.%, vorzugsweise 50 - 200 Gew.%, bezogen auf das rohe α-Bisabolol eingesetzt.

Das verwendete Austauscherharz ist vorzugsweise ein hochpolymeres vernetztes Polykondensations- oder Polymerisationsharz in Gelform, dessen aktive Gruppen wahlweise saure Gruppen, vorzugsweise Sulfonsäure-, Carboxylat-oder Phenolgruppen oder basische Gruppen, vorzugsweise ein-oder mehrfach alkylierte Aminogruppen sind. Bei Bedarf können dabei auch unterschiedliche Harze in Mischung miteinander oder hintereinandergeschaltet zum Einsatz kommen. Als Einsatzmenge sind in der Regel 5 - 100 Gew.%, vorzugsweise 10 - 50 Gew.% Harz, bezogen auf das rohe α-Bisabolol ausreichend.

Nach erfolgtem Durchlauf durch das Austauscherharz und anschließender Abtrennung des Lösungsmittels kann das α-Bisabolol noch weiteren üblichen Reinigungsoperationen unterworfen werden, um auch noch andere, geruchsneutrale Begleitstoffe abzutrennen und so zu einer stärkeren Anreicherung zu kommen. Diese weiteren Reinigungsoperationen können z.B. in einer Adsorptions-Behandlung mit Aktivkohle bestehen und/oder in einer Destillation unter vermindertem Druck. Ebenso können auch Extraktionsverfahren oder chromatographische Verfahren in Betracht kommen, wenn die Art der abzutrennenden geruchsneutralen Begleitstoffe dies erfordert. Andererseits kann auch auf eine Nachbehandlung mit weiteren üblichen Reinigungsoperationen ganz verzichtet werden, wenn keine Abtrennung anderer, geruchsneutraler Begleitstoffe nach erfolgter Abtrennung der Geruchsstoffe mehr notwendig ist.

Das erfindungsgemäße Verfahren zeichnet sich aus durch die Verwendung sehr einfacher Apparaturen, einen geringen Arbeits-und Hilfsstoffaufwand und eine sehr niedrige Umweltbelastung. Es kann für die Reinigung von reinen Bisabolol-Stereoisomeren, teilweisen racemischen Gemischen sowie optisch inaktiven Gemischen natürlichen oder synthetischen Ursprungs verwendet werden.

Nachfolgend wird die Erfindung in einem Ausführungsbeispiel (welches die Erfindung erläutern soll, ohne sie einzugrenzen) beschrieben und mit zwei Beispielen nach dem Stand der Technik verglichen. In allen Fällen wurde dabei von rohem Candeia-Öl ausgegangen, einem unangenehm riechenden ätherischen Öl aus *Vanillosmopsis erythropappa* mit einem Gehalt von 81,4 Gew.% (-)-α-Bisabolol nach Gaschromatogramm.

**Beispiel 1 (Vergleichsbeispiel)**

In Analogie zu Beispiel 1 der DE-PS 23 17 583 wurden 100 g Candeia-Öl in 1000 ml Petrolether (Siedebereich 50 - 70 °C) gelöst. Die Lösung wurde im Scheidetrichter 500 ml mit 5 %iger wäßriger Natronlauge (= 26,3 Gew.% NaOH, bezogen auf das eingesetzte Candeia-Öl) während der nächsten 24 h mehrmals intensiv geschüttelt. Dann wurde die wäßrige Phase abgetrennt und die organische Phase dreimal mit je 100 ml Wasser gewaschen und über Natriumsulfat getrocknet. Danach wurde der Petrolether im Vakuum (50 Torr, Badtemperatur 50 °C) abdestilliert. Es fielen 91,0 g Öl an, das anschließend an einer Glasfüllkörperkolonne (wirksame Länge: 60 cm) unter vermindertem Druck destilliert wurde. Die bei 96 - 104 °C (0,13 mbar) übergehenden Anteile wurden gesammelt und bildeten das Endprodukt.

Ausbeute: 51,0 g (51 %, bezogen auf das eingesetzte Candeia-Öl)
Reinheit: 96,5 % α-Bisabolol, 2,3 % Bisabololoxid B gemäß GC
GC: (DB-Wax-30 N, 30 m, 200 °C)

**Beispiel 2 (Vergleichsbeispiel)**

In Analogie zu Beispiel 3 der DE-PS 23 17 583 wurden 100 g Candeia-Öl in 1000 ml Petrolether (Siedebereich 50 - 70 °C) gelöst. Die Lösung wurde 1 h mit 25 g Kaliumcarbonat (fest) und 25 g Aktivkohle geschüttelt. Nach Filtration wurde der Rückstand viermal mit je 75 ml Petrolether (Siedebereich 50 - 70 °C) ausgewaschen, und die vereinigten organischen Phasen wurden im Vakuum (50 Torr, Badtemperatur 50 °C) eingeengt. Es fielen 85,1 g Öl an, das anschließend an einer Glasfüllkörperkolonne (wirksame Länge: 60 cm) unter vermindertem Druck fraktioniert wurde. Die bei 95 - 98 °C (0,13 mbar) übergehenden Anteile wurden gesammelt und bildeten das Endprodukt.

Ausbeute: 52,0 g (52 %, bezogen auf das eingesetzte Candeia-Öl)
Reinheit: 96,5 % α-Bisabolol, 2,0 % Bisabololoxid B nach GC
GC: Bedingungen wie Beispiel 1

**Beispiel 3 (Erfindungsbeispiel)**

Für die erfindungsgemäße Behandlung des zugrundeliegenden Candeia-Öl hat sich ein basisches Austauscherharz als besonders geeignet erwiesen. Bevorzugt wird Amberlyst A-26 (geschütztes Warenzeichen) eingesetzt, ein makroporöser Anionenaustauscher in der OH-Form.

100 g Candeia-Öl wurden in 100 g Isopropanol gelöst. Diese Lösung wurde innerhalb von 2 h über 40 g Amerlyst A-26 (Austauschersäule: Länge 30 cm, Durchmesser 2,5 cm) gegeben und anschließend wurde das Harz noch innerhalb von 30 min mit 100 g Isopropanol gespült. Die vereinigten Elutionen wurden dann unter vermindertem Druck vom Lösungsmittel befreit. Das so erhaltene Produkt (89 g gelbes Öl) wurde anschließend an einer Vigreux-Kolonne (Glas, wirksame Länge: 25 cm) unter vermindertem Druck andestilliert, um zunächst die leicht flüchtigen Bestandteile abzutrennen. Die dabei bis 108 °C/0,27 mbar übergehenden Anteile (23,8 g), die u.a. α-und β-Bisabolene) enthalten, wurden zur anderweitigen Verwendung gesammelt. Danach wurde die Andestillation abgebrochen und der das α-Bisabolol enthaltende Sumpf innerhalb von 20 min unter vermindertem Druck über eine Claisen-Brücke destilliert. Die bei 110 - 122 °C/0,27 mbar übergehenden Anteile wurden gesammelt und bildeten das Endprodukt.

Ausbeute: 53,0 g (53 %, bezogen auf das eingesetzte Candeia-Öl).
Reinheit: 97,9 % (-)-α-Bisabolol, 1,8 % Bisabololoxid B nach GC
GC: Bedingungen wie Beispiel 1

Das Endprodukt ist ein nahezu farbloses Öl, welches nur noch den schwach blumigen Eigengeruch des (-)-α-Bisabolos besitzt.

Das in allen Fällen im Endprodukt noch enthaltene Bisabololoxid B ist ein farblich und geruchlich nicht störender Begleitstoff, der sich destillativ nicht vom α-Bisabolol trennen läßt. Dieser Begleitstoff ist überhaupt nur mit hochauflösenden gaschromatischen Methoden erkennbar und wird deshalb oftmals mit als α-Bisabolol gerechnet.

**Ansprüche**

1. Verfahren zur Reinigung von α-Bisabolol, welches als Rohprodukt durch Synthese oder durch Isolierung aus Pflanzenmaterial gewonnen wurde, dadurch gekennzeichnet, daß eine Lösung des rohen α-Bisabolols in einem inerten Lösungsmittel mit einem Siedepunkt zwischen 40 und 200 °C, vorzugsweise 60 - 150 °C, über ein Austauscherharz filtriert, anschließend vom Lösungsmittel befreit und dann ggfs. weiteren Reinigungsoperationen unterworfen wird.

2. Verfahren nach Anspruch 1, dadurch gekennzeichnet, daß als Lösungsmittel ein aliphatischer Alkohol mit bis zu 6 C-Atomen, vorzugsweise Isopropanol, verwendet wird.

3. Verfahren nach Anspruch 1 oder 2, dadurch gekennzeichnet, daß das Lösungsmittel in einer Menge von 10 bis 1000 Gew.%, vorzugsweise 50 - 200 Gew.%, bezogen auf das rohe α-Bisabolol, eingesetzt wird.

4. Verfahren nach einem der Ansprüche 1 bis 3, dadurch gekennzeichnet, daß als Austauscherharz ein hochpolymeres vernetztes Polykondensatations-oder Polymerisationsharz in Gelform verwendet wird, dessen aktive Gruppen wahlweise saure Gruppen, vorzugsweise Sulfonsäure-, Carboxylat-oder Phenolgruppen oder basische Gruppen, vorzugsweise ein-oder mehrfach alkylierte Aminogruppen sind.

5. Verfahren nach einem der Ansprüche 1 bis 4, dadurch gekennzeichnet, daß das Austauscherharz in einer Menge von 5 -100 Gew.%, vorzugsweise 10 - 50 Gew.%, bezogen auf das rohe α-Bisabolol eingesetzt wird.

Europäisches Patentamt

# EUROPÄISCHER RECHERCHENBERICHT

Nummer der Anmeldung

EP 86115246.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | DE - C3 - 2 317 583 (DEUTSCHE GOLD- UND SILBER)<br>* Anspruch 1 *<br>-- | 1 | C 07 C 33/14<br>C 07 C 29/76 |
| A | EP - A2 - 0 125 833 (DUOLITE INTERNATIONAL)<br>* Zusammenfassung *<br>---- | 1 | |

| RECHERCHIERTE SACHGEBIETE (Int. Cl.4) |
|---|
| C 07 C 33/00<br>C 07 C 29/00 |

Der vorliegende Recherchenbericht wurde für alle Patentansprüche erstellt.

| Recherchenort | Abschlußdatum der Recherche | Prüfer |
|---|---|---|
| WIEN | 01-06-1987 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82